Europäisches Patentamt

European Patent Office

Office européen des brevets

(11) Numéro de publication: **0 139 548**
**B1**

(12) **FASCICULE DE BREVET EUROPEEN**

(45) Date de publication du fascicule du brevet: **12.10.88**

(21) Numéro de dépôt: **84401634.5**

(22) Date de dépôt: **06.08.84**

(51) Int. Cl.⁴: **A 61 M 1/10**

(54) **Dispositif de régulation d'une pompe.**

(30) Priorité: **08.08.83 FR 8313236**

(43) Date de publication de la demande:
**02.05.85 Bulletin 85/18**

(45) Mention de la délivrance du brevet:
**12.10.88 Bulletin 88/41**

(84) Etats contractants désignés:
**AT BE CH DE FR GB IT LI NL SE**

(56) Documents cités:
**EP-A-0 030 760**
**DE-A-2 734 075**
**DE-A-2 740 062**
**FR-A-2 244 546**

(73) Titulaire: **UNIVERSITE DU DROIT ET DE LA SANTE LILLE II**
**42, rue Paul Duez**
**F-59800 Lille (FR)**

(72) Inventeur: **Pieronne, Alain**
**Résidence de Stael 63, rue d'Isly**
**F-59000 Lille (FR)**
Inventeur: **Logier, Régis**
**19, rue des Liliums**
**F-59134 Herlies (FR)**
Inventeur: **Delecroix, Michel**
**Résidence des Peupliers 2, rue d'Haubourdin**
**F-59155 Faches Thumesnil (FR)**
Inventeur: **Soots, Georges**
**17, rue Bazinghien**
**F-59000 Lille (FR)**

(74) Mandataire: **Ecrepont, Robert Pierre**
**12 Place Simon Vollant**
**F-59800 Lille (FR)**

EP 0 139 548 B1

Courier Press, Leamington Spa, England.

# Description

L'invention se rapporte à un dispositif de régulation de la vitesse d'une pompe d'un système d'assistance d'un organe vivant et au système d'assistance d'un organe vivant pourvu de ce dispositif.

Elle s'applique au domaine médical, plus particulièrement mais non exclusivement, à la régulation du circuit de dérivation d'un système "left-vent" ou d'un rein artificiel ou plus spécialement d'un système d'assistance gauche éventuellement couplée à une assistance droite et ce, que le débit dans ce circuit soit continu ou pulsé.

Si l'intérêt des dérivations ventriculaires gauches est apparu très tôt comme plein de promesses dans le traitement du choc cardiogénique grave, les techniques actuelles, sont, en général, insuffisantes pour traiter les chocs cardiogéniques consécutifs à une incompétence myocardique massive rencontrés en "post-circulation extra-corporelle" communément dénommée "POST-C.E.C".

De manière connue (EP—A—30760, page 1 lignes 6 à 9), cette circulation extracorportelle (C.E.C) a pour rôle d'assurer la fonction du coeur et du poumon pendant leur exclusion au cours d'opérations chirurgicales intra-cardiaques.

Après cette exclusion, seule une assistance gauche, éventuellement associée à une assistance droite peut assurer la perfusion des tissus périphériques réalisant un équilibre favorable à une éventuelle récupération du myocarde.

Les effets bénéfiques de ce nouvel équilibre entre les apports et la demande énergétique ont été démontrés chez l'animal.

C'est en effet, la décharge complète du ventricule gauche, auquel se substitue le dispositif d'assistance, qui permet l'amélioration des caractéristiques de perfusion du myocarde. Par l'équilibre ainsi obtenu, on réalise alors:

— une perfusion coronaire de niveau élevé,

— une diminution du volume du ventricule et de la pression sous endocardique et,

— une réduction de la consommation d'oxygéne du fait qu'aucun travail externe n'est réalisé par le coeur.

L'assistance gauche s'adresse donc à des malades sont l'état myocardique ne permet pas le sevrage de la "C.E.C" malgré l'utilisation des catécholamines et de la contre-pulsion intra-aortique.

Une assistance gauche de plusieurs jours permet alors au myocarde de récupérer.

Une telle assistance de plusieurs jours a également permis de sevrer cinquante pour cent des malades de ce type.

Le débit dans le circuit peut être continu mais dans l'état actuel des connaissances, il semble important d'assurer un débit pulsé et ce pour plusieurs raisons telles que:

— atténuation de la réponse au stress de la circulation extra-corporelle (C.E.C) par diminution de la libération de catécholamines avec pour conséquence:

— diminution des résistances vasculaires systémiques et donc diminution du travail cardiaque,

— diminution des libérations hormonales hypophysaires surrénaliennes.

Le système classique de C.E.C comprend (EP—30760):

— des moyens de conduite du sang comportant un conduit amont de prélèvement du sang du malade et un conduit aval d'injection du sang vers le malade,

— des moyens de pompage engendrant une circulation du sang du conduit amont de prélèvement vers le conduit aval d'injection,

— une unité d'oxygénation du sang placée entre le conduit amont de prélèvement et le conduit aval d'injection et,

— un dispositif de régulation du débit de sang délivré vers un malade,
lequel dispositif de régulation comprend:

— des moyens de détection de la quantité de sang contenue à chaque instant dans l'unité d'oxygénation, adaptés pour délivrer un signal électrique représentatif à chaque instant de cette quantité de sang,

— une mémoire dite de consigne recevant le signal issu des moyens de détection et associée à une logique de blocage permettant de figer à un instant donné le signal reçu dans la mémoire,

— un comparateur recevant, d'une part, le signal issu des moyens de détection, d'autre part, le signal issu de la mémoire de consigne et adapté pour délivrer un signal d'écart représentatif de la différence entre les signaux reçus,

— un ensemble potentiométrique adapté pour délivrer un signal de vitesse correspondant à une vitesse initiale désirée des moyens de pompage.

— un sommateur recevant le signal d'écart issu du comparateur et le signal de vitesse issu de l'ensemble potentiométrique et adapté pour délivrer vers les moyens de pompage, un signal d'asservissement approprié pour accélérer les dits moyens de pompage en cas d'augmentation de la quantité de sang détectée et pour ralentir ces moyens de pompage en cas de baisse de la quantité de sang détectée.

S'il présente des analogies, le circuit d'assistance gauche objet de l'invention ne fait pas appel aux mêmes paramètres et, de ce fait, sa régulation en fonction de la quantité de sang ne peut convenir.

La régulation nécessite évidemment un monitorage de la pression auriculaire gauche di-après dite "P.O.G" car les variations notamment brusques de débit, de pression et de volémie, seraient mal supportées par les patients en choc cardiogénique.

De plus, si la pression devient négative, par l'effet de succion qui se produirait, apparaîtrait un risque de collapsus des veines pulmonaires et un risque d'embolie gazeuse. L'intérêt d'une régulation automatique est d'autant plus important que ces circuits sont généralement mis en place pour plusieurs jours.

Il faut pendant tout ce temps surveiller le circuit pour écarter tous dangers tels que hyperpression, rupture de ligne, colmatage du filtre, embolie gazeuse.

Il est donc difficilement concevable de les mettre en oeuvre sans s'assurer de sécurité, en particulier, d'une régulation automatique et d'alarmes.

Dans le but d'assurer un débit régulier mais aussi donc dans le but d'éviter les accidents évoqués ci-dessus, un résultat que l'invention vise à obtenir est un dispositif qui régule le débit dans l'organisme et donc qui, dans l'exemple choisi, régule les dérivations ventriculaires gauches et qui, au besoin, peut également piloter un circuit de dérivation ventriculaire droite simultanée.

A cet effet, elle a pour objet un dispositif régulateur de la vitesse d'au moins d'une pompe d'un système d'assistance d'un organe vivant comprenant:

— des moyens de mesure et d'enregistrement des conditions réelles de fonctionnement,

— des moyens d'élaboration et d'enregistrement des valeurs des consignes correspondantes de fonctionnement,

— des moyens de comparaison de chacune des valeurs réelles à la valeur de consigne correspondante et d'élaboration d'un signal proportionnel à l'écart entre ces valeurs,

— des moyens d'élaboration, en fonction de ce signal proportionnel du signal déterminant la vitesse de la pompe,

— des portes de contrôle du seuil de chacun de ces signaux proportionnels et,

— des alarmes commandées chacune par l'un des signaux proportionnels lors de leur franchissement du seuil fixé.

L'invention est notamment caractérisée en ce qu'au moins l'un des moyens de mesure des conditions réelles de fonctionnement consiste en un moyen de mesure de pression.

L'invention concerne également le système d'assistance pourvu de ce dispositif.

L'invention sera bien comprise à l'aide de la description ci-après faite, à titre d'exemple non limitatif, d'un circuit d'assistance gauche, et ce, en regard du dessin ci-annexé qui représente schématiquement:

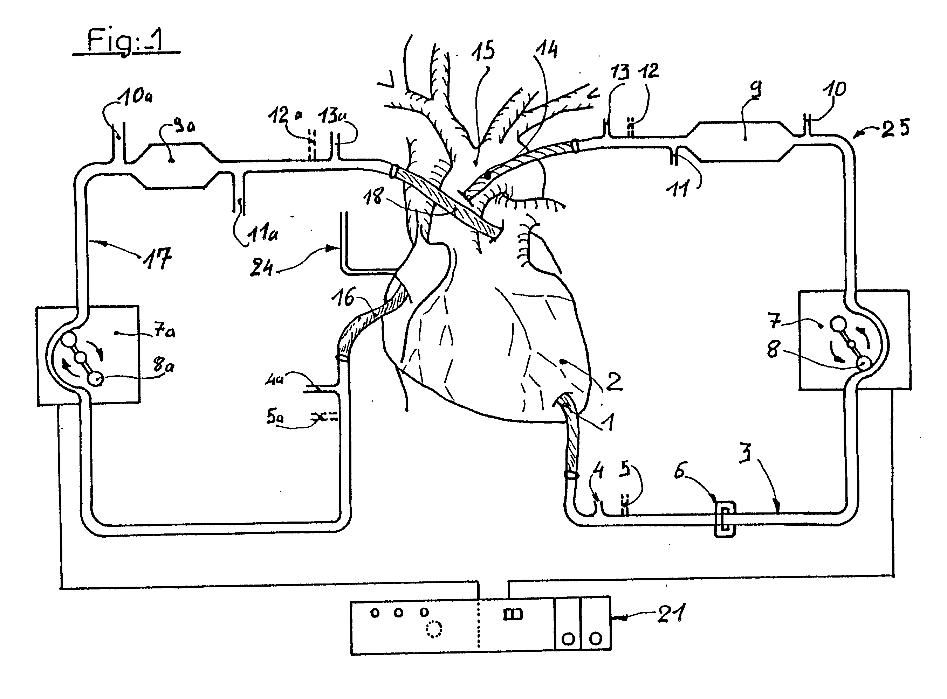

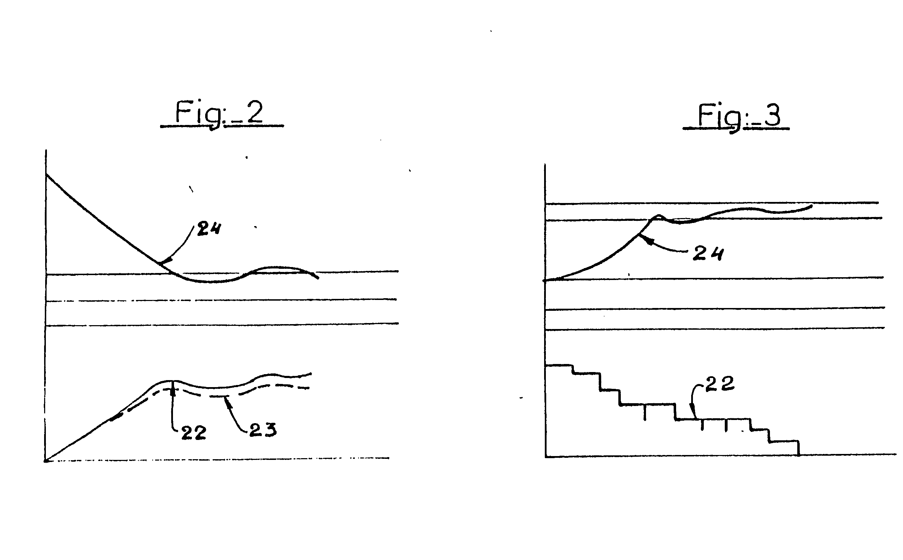

— figure 1: un circuit d'assistance,

— figure 2: les courbes du débit et de la P.O.G lors de la mise en route de l'assistance,

— figure 3: les courbes du débit et de la P.O.G lors du sevrage.

— figure 4: le synoptique général d'un dispositif,

— figure 5: le détail des entrées du dispositif,

— figure 6: le détail des sorties du dispositif,

— figure 7: le détail du calculateur du dispositif,

— figure 8: une variante de réalisation de la commande de la pompe en cas de débit pulsé.

En se reportant au dessin, on voit que le circuit d'assistance gauche, de manière comparable à celui d'une C.E.C, comprend une canule 1 suturée à la sortie de l'organe telle le niveau de la pointe du ventricule gauche 2 ou le niveau de l'oreillette gauche et qui assure une décharge de cet organe.

Par un raccord non métallique (non représenté) elle est connectée à l'entrée d'un circuit 3, par exemple en chlorure de polyvynil.

Ce circuit, dit "ligne artérielle", passe tout d'abord par une purge d'air 4 puis par une éventuelle entrée 5 d'un produit, tel de l'héparine, et un détecteur de bulles 6 avant d'arriver dans une pompe 7 à galets rotatifs 8 dont la vitesse est réglable selon la différence de potentiel appliquée à ses bornes.

Il s'agira par exemple d'une pompe d'un modèle couramment utilisé pour la circulation extra-corporelle.

En sortie de la pompe 7, la ligne artérielle passe par un filtre 9 en amont et en aval duquel sont prévues deux sorties 10, 11 qui permettent des prises de pression de part et d'autre du filtre 9 qui lui-même est interchangeable.

Avant d'arriver à une seconde canule 14 destinée à la réinjection dans l'entrée de l'organe par exemple au niveau de l'aorte 15, le circuit 3 se poursuit en passant éventuellement par un système sans héparine ou par une entrée 12 de produit, tel de la protamine, puis par une nouvelle purge d'air 13.

Quant à l'éventuel circuit d'assistance droite, elle est semblable à la précédente puisqu'elle comprend par exemple, tout d'abord une canule 16 suturée à la sortie de l'organe, tel les veines caves, et qui, par un raccord non métallique est raccordée à un circuit 17 passant tout d'abord par une purge d'air $4_a$ et une eventuelle entrée $5_a$ de produit tel de l'héparine avant de parvenir à une pompe $6_a$ puis à un filtre $9_a$ en amont et en aval duquel des entrées $10_a$, $11_a$ permettent des prises de pression.

En sortie du filtre $9_a$, après passsage par une eventuelle entrée $12_a$ de produit, tel de la protamine, et une seconde purge d'air $13_a$, le circuit est raccordé à une canule pulmonaire 18.

Au détecteur de bulles 6 du circuit d'assistance gauche, sont raccordés des moyens qui arrêtent la pompe 7 en cas de détection de bulles.

Sa position en amont de la pompe 7 permet, malgré le temps de latence de la dite pompe 7 de supprimer le risque d'injection de bulles dans le circuit.

Comme indiqué plus haut, la pompe 7, $7_a$ est d'un type dont le débit est lié à la différence de potentiel appliquée à ses bornes.

Afin de régler cette différence de potentiel en fonction des informations prélevées sur ce circuit, le circuit comprend un dispositif régulateur 21.

Ce dispositif fonctionne notamment par référence à la pression réelle dans l'organe, pression ci-après dite "P.O.G", et à une pression souhaitée dite pression de consigne ou préprogrammée.

De ce fait, tout d'abord, il comprend des moyens d'enregistrement d'une pression de consigne et de mesure 24 de la pression réelle.

En se reportant figure 2, on voit que, en deux ou trois minutes, lors de la mise en route de l'assistance, tant le débit 22 dans le circuit gauche 3, que celui 23 dans l'éventuel circuit droit 17 augmentent selon une pente prédéterminée pour arriver à un débit optimum.

Pendant ce temps, la pression réelle 24 dans l'organe (P.O.G) diminue symétriquement au débit.

Dès que la pression préprogrammée est atteinte, entre alors en jeu le dispositif selon l'invention qui, à cet effet, comprend des moyens comparant la P.O.G et la pression préprogrammée qui passe par deux ports tels que:

— si la P.O.G chute d'une certaine valeur au dessous de la valeur donnée, le dispositif diminue le débit pour tendre à majorer la P.O.G tant que celle-ci restera inférieure à la consigne et,

— si la P.O.G remonte au-dessus de la valeur, le dispositif réaugmente le débit pour abaisser la P.O.G dans les mêmes proportions et ceci jusqu'à l'obtention du débit qui correspond à la pression préprogrammée.

Le ports d'alarmes sont tels que si le signal de P.O.G. réelle chute d'une valeur inférieure à une valeur donnée (par exemple cinq millimètres de mercure), ils provoquent une alarme sonore et un arrêt de pompe.

Pour reprendre l'opération, cette pompe devra alors être réarmée manuellement et l'on se retrouvera dans le cas de figure évoquée plus haut de la mise en route du circuit d'assistance.

Dans le cas de l'assistance cardique gauche, le coeur droit fonctionnant, la pompe étant arrêtée et le débit étant donc nul, la P.O.G doit remonter.

Ce n'est que si, avec l'assistance gauche seule, l'index cardiaque optimum n'est pas réalisé et s'il existe une pression auriculaire gauche basse avec pression veineuse élevée ou une pression artérielle en oxygéne inférieure ou égale à soixante millimètres de mercure, qu'une assistance droite complémentaire doit être envisagée.

Le dispositif de régulation 21 comprend en outre des moyens de comparaison de la pression mesurée dans la sortie 11 en aval du filtre 9 par rapport à une valeur de pression donnée préprogrammée, différente de la pression de consigne, de même que des moyens de comparaison des pressions dans les sorties 10, 11 amont et aval du dit filtre, la différence de ces pression provoque l'alarme colmatrage de filtre.

En combinaison avec une coudure 25 dans la partie du circuit situé en aval de la pompe 7, les premiers moyens détectent toute hyperpression.

En cas de dépassement d'un seuil fixé, ceci commande une alarme sonore et une alarme visuelle indiquant le type de problème qui provoque l'arrêt de la pompe.

Quand cette différence atteint une valeur donnée préprogrammée, les dits moyens actionnent alors les alarmes sonores et visuelles indiquant ce type de problème.

Dans une variante de réalisation, le dit dispositif comprend également un moyen d'évaluation semi-quantitative de bulles avec contrôle auditif.

Le dispositif comprend encore un système de sevrage par décrémentation du débit d'assistance par exemple d'environ cent millilitres toutes les heures.

Pour le sevrage, pour tenir compte de l'état myocardique, la pression de consigne sera alors préprogrammée à un niveau assez élevé puis:

— le débit sera décrémenté, tant qu'avec le débit inférieur ainsi obtenu, la pression préprogrammée n'est pas dépassée, et cette décrémentation pourra se poursuivre jusqu'à ce que le sevrage soit complet,

— si la pression préprogrammée est dépassée les moyens précités assureront d'une part, immédiatement, l'arrêt de la décrémentation et le retour au débit immédiatement antérieur ainsi que la mise en route d'une alarme visuelle et sonore et d'autre part, au bout d'un certain temps par exemple l'heure suivante, l'engagement d'une nouvelle tentative de décrémentation.

Le dispositif pourra également comprendre une touche "réset" permettant d'arrêter l'alarme sonore, l'alarme visuelle restant quant à elle allumée tant que le défaut persistera.

Si ce défaut se produit plusieurs fois, deux solutions pourront alors être envisagées:

— soit continuer l'assistance en arrêtant manuellement la tentative de sevrage,

— soit choisir un autre niveau de pression de consigne permettant à la décrémentation de reprendre.

Le maintien du débit d'assistance à un niveau voulu (figure 2) peut évidemment se faire en variant le niveau de la P.O.G par d'autres moyens et par exemple:

— en jouant sur la post-charge à l'aide de vasodilatateurs (nitroprussiate de soude),

— en jouant sur la pré-charge par le "remplissage" du malade (transfusion).

Ces malades, dont le saignement est important au début de ce type d'assistance, ont, bien entendu, une régulation de la pression veineuse centrale (deuxième pression). Cette pression n'est pas entrée dans le présent dispositif pour assurer la régulation, mais sa valeur est néanmoins prise en compte pour déterminer des alarmes.

Dans le cas évoqué plus haut où, l'adjonction d'une assistance droite est nécessaire, le débit de la pompe corespondante $7_a$ sera régulé par le même dispositif 21 que celui de la pompe 7 de l'assistance gauche.

A cet effet, les débits étant physiologiquement identiques, à un ou deux pour cent près (moins dix pour cent en assistance couplée) le dispositif comprendra simplement un moyen, tel un potentiomètre, permettant de faire varier, dans des proportions suffisantes, le débit droit par rapport au débit gauche, par l'ajustement de la différence de potentiel appliquée aux bornes de la pompe droite $7_a$ par rapport à celle de la pompe gauche 7, et ce, par exemple de plus dit pour cent à moins quinze pour cent.

En début d'assistance, ces moyens assureront une différence nulle puis le réglage se fera en fonction de l'adaptation d'un coeur par rapport à l'autre.

Dans une variante de réalisation, les moyens faisant varier cette différence de potentiel et donc de débit sont pilotés par la P.O.G.

Ce réglage est bien sûr inutile si la contractilité restante assure à elle seule une régulation.

Comme indiqué plus haut la régulation du débit de pompe se fait à l'aide d'une tension continue que l'on fait varier, par exemple, de zéro à quinze volts.

Dans le cas où un débit pulsé est souhaité, le dispositif est pourvu de moyens de surmodulation de cette tension.

Le patient etant bien sûr, dans tous les cas, sous la surveillance constante d'un électrocardiographe, le dispositif peut comprendre des moyens tels que l'électrocardiogramme obtenu puisse avantageusement être rentré dans le régulateur qui comprend alors d'une part des moyens de mise en forme de l'espace inter-systolique électrique, dit "espace R.R" et d'autre part deux potentiomètres 26, 27 permettant de déterminer, par rapport à l'espace R.R précédent, la durée, le moment et l'intensité de l'accélération de la pompe, et ce, indépendamment de tout autre facteur.

Le début d'impulsion se fera entre zéro et soixante pour cent du temps R.R., la fin d'impulsion entre quarante et cent pour cent du temps R.R.

Selon le modèle de pompe utilisé, l'amplitude est réglée pour permettre une éjection diastolique de vingt à quarante millilitres.

Pour des raisons de facilité de mise en oeuvre et de mise au point, l'appareil régulateur 21 a été construit autour d'un microprocesseur de type dit "Z80".

Il est constitué de trois blocs principaux 28, 29, 30 à savoir:

— un bloc 28 de mise en forme des signaux d'entrée,

— un bloc 29 calculateur,

— un bloc 30 de mise en forme des signaux de sortie.

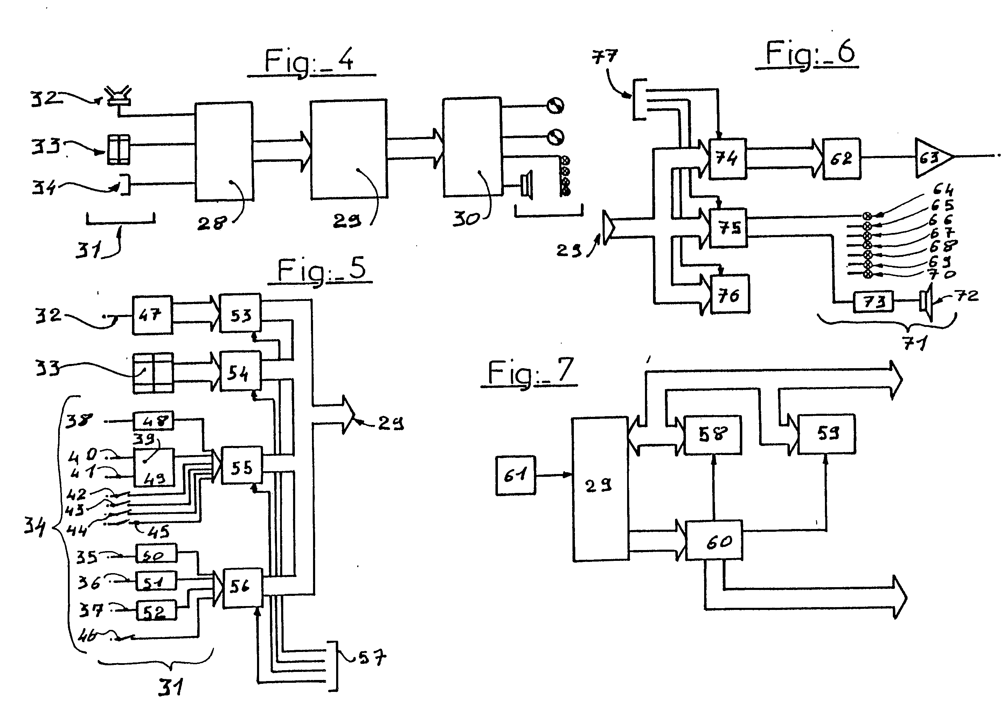

Les organes d'entrée 31 (figures 4 et 5) du bloc 28 de mise en forme des signaux d'entrée comprennent principalement un moyen 32 d'entrée de la P.O.G prise par le moyen 24, des roues codeuses 33 et des entrées 34 en provenance d'organes de sécurité.

Pour utiliser la P.O.G, il est nécessaire que le bloc 28 comprenne un moyen 32 de transformation de la P.O.G en tension électrique et que ce moyen 32 soit tel qu'il donne non seulement la tension représentant la pression instantanée mais également les tensions correspondant aux alarmes haute et basse de la P.O.G.

Les roues codeuses 33 donneront au calculateur 29 la valeur de P.O.G à atteindre (pression de consigne) pour lui permettre d'adapter en conséquence le débit.

Les entrées 34 en provenance d'organes de sécurité seront de deux types principaux comprenant, l'un, celles commandant l'arrêt de la pompe, l'autre, celles commandant des alarmes dites "normales".

Trois entrées commandent l'arrêt de la pompe:

— l'entrée 35 correspondant au signal de la P.O.G inférieure à une limite fixée,

— l'entrée 36 du signal issu du capteur de pression post-filtre qui, en combinaison avec le circuit coudé, détecte une haute pression dans le circuit d'assistance,

— l'entrée 37 du signal issu du détecteur de bulles 6 adapté sur le circuit d'assistance.

Les entrées commandant des alarmes normales comprennent l'entrée 38 du signal issu du module de pression et indiquant une P.O.G supérieure à une limite fixée et l'entrée du signal 39 de la différence de pression calculée à partir des pressions 40 et 41 venant des entrées 10, 11 pré et post-filtre du circuit et signalant si le filtre 9 est colmaté.

Les organes d'entrée divers 31 comprennent encore des moyens tels:

— un bouton de réarmement 42, il permet de réarmer le circuit d'assistance en cas d'arrêt sur alarme,

— un inverseur 43 sevrage-régulation: il permet de choisir entre la fonction de régulation et la fonction de sevrage,

— un bouton 44 d'arrêt des alarmes sonores,

— un bouton 45 d'extinction des voyants d'alarme,

— un bouton 46 d'arrêt manuel de la pompe: il permet à l'utilisateur d'arrêter la pompe quant il le désire, et,

— deux potentiomètres linéaires 26, 27 permettant, d'une part, de faire varier le moment d'accélération de pompe par rapport à l'espace R.R de zéro à soixante pour cent, et, d'autre part, de faire varier le moment d'arrêt de cette accélération de pompe par rapport à l'espace R.R de quarante à cent pour cent.

Lors d'un débit pulsé, les entrées du dispositif comprennent encore des moyens (non représentés) tels que:

— un bouton "marche/arrêt" du système pulsé,

— un potentiomètre de réglage du volume pulsé,

— un bouton de réglage de l'amplitude des pulsations par exemple à l'aide d'une bascule telle un trigger.

Le circuit 28 de mise en forme des signaux d'entrée (figure 4) comprend quant à lui:

— des moyens 47 transformant en signaux logiques les signaux analogiques que sont les grandeurs d'entrée et ce, afin qu'ils puissent être traités par le calculateur 29 et,

— des moyens 48 à 51 de mise à niveau des signaux 52 de détection de seuil.

Une fois, ces signaux traités, ils pénètrent dans le calculateur 29 via des ports d'entrée 53 à 56, dont le microprocesseur possède à cet effet les commandes 57.

Comme indiqué plus haut, dans le cas d'une assistance à la fois gauche et droite, la régulation des deux circuits se fait néanmoins par rapport à la même P.O.G.

Les divers signaux d'alarmes provenant du circuit droit seront quand même pris en compte et à cet effet, le dispositif comprendra les entrées correspondantes qui seront, de préférence, réparties sur des ports différents à savoir sur un port 55 pour les entrées commandant des alarmes "normales" et sur un port 56 pour les entrées commandant l'arrêt de la pompe 7, 7a.

Les traitements de ces différents signaux du circuit droit étant les mêmes que pour les signaux du circuit gauche, ils ne sont pas représentés sur le synoptique.

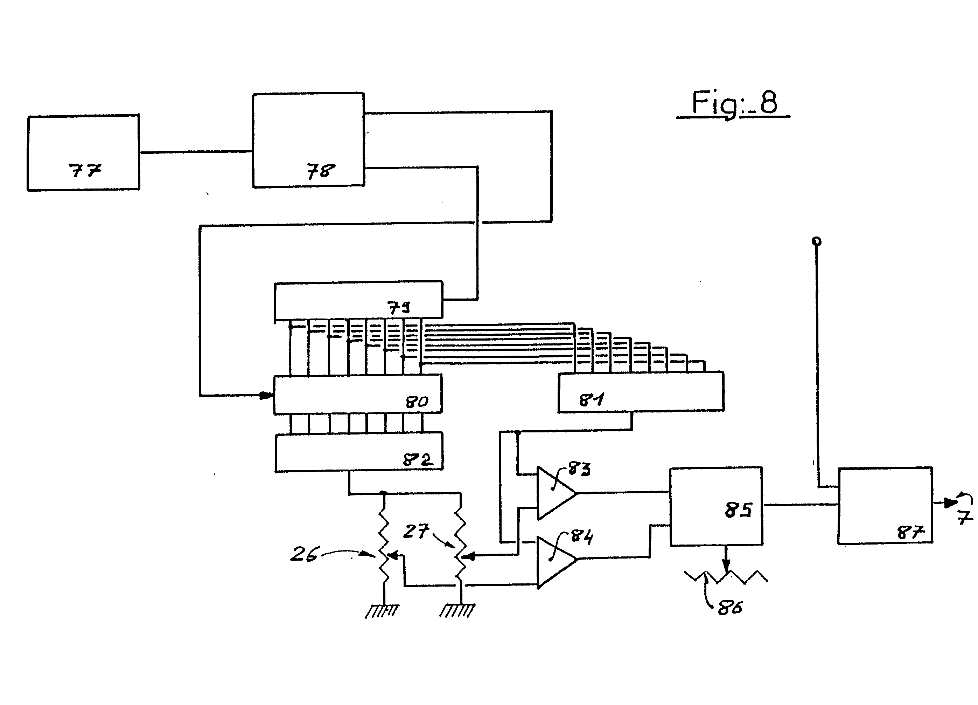

Comme déjà spécifié, le calculateur 29 (figure 7) est basé sur un microprocesseur du type "Z 80". A ce microprocesseur sont adjoints:

— une mémoire morte 58 pour les programmes et,

— une mémoire vive 59 pour les données ainsi que,

— des circuits logiques ordinaires 60 réalisant le décodage des adresses mémoires et des adresses périphériques et,

— une horloge 61 pilotée par quartz.

Après traitement des informations d'entrée le calculateur 29 délivre, entre-autres signaux, une commande qui est destinée à la pompe mais qui est de type numérique et qu'il faut au préalable transformer en tension électrique.

En effet, la pompe 7, 7a utilise, pour être régulée, une tension continue ou modulée comprise entre zéro et quinze volts.

C'est le rôle du circuit 30 de mise en forme des signaux de sortie (figures 4 et 6) qui comprend donc des moyens de conversion digital-analogique 62 et de mise à niveau 63.

Le circuit de sortie 30 comprend encore des moyens 64 à 70 de visulisation des alarmes tels des diodes électro-luminescentes et une alarme sonore 71 tel un haut parleur 72 excité par un oscilleur basse fréquence 73.

Comme pour les informations d'entrée, toutes ces informations de sortie sont issues du calculateur 29 via des ports de sortie 74, 75, 76 dont le micro-processeur 29 possède à cet effet les commandes 77.

Dans le cas d'une assistance à la fois gauche et droite, le dispositif comprend un amplificateur opérationnel à gain variable commandant à partir du signal de la pompe gauche 7, la pompe 7a du circuit droit.

Ainsi, la vitesse de la pompe droite par rapport à la pompe gauche sera réglable manuellement par ajustement de ce gain.

En cas d'anomalie soit du circuit gauche, soit du circuit droit, le port 74 commande éventuellement l'arrêt des deux pompes alors que les alarmes du circuit gauche et/ou droit (non représentées) sont commandées au travers des ports 75, 76 et du calculateur 29.

Dans le cas d'un débit pulsé, l'ensemble comprend:

— un circuit 77 de mise en forme de l'onde,

— un circuit 78 de détection tant du front descendant, assurant la remise à zéro du compteur 79, que du front montant, assurant la mémorisation du compteur 80,

— un compteur analogique 81 qui permet de mesurer le temps R.R qui est inversé et transformé en différence de potentiel par un analyseur digital 82,

— deux potentiomètres linéaires 26, 27 qui permettent de moduler le temps d'accélération ou de décélération de la pompe 7,

— un deuxième analyseur digital commandé par le même compteur 81,

— deux amplificateurs opérationnels 83, 84 qui recoivent ces courants modulés ainsi que les signaux du deuxième analyseur digital.

On obtient donc par rapport à l'espace R.R, le moment de montée en accélération et le moment de retour à la normale.

Les signaux passent alors dans un moyen de mise en forme 85 puis par un potentiomètre 86 de mise à niveau du volume pulsé avant d'arriver à leur mélangeur 87 avec le signal venant du microprocesseur et appelé tension pompe.

Bien entendu, un autre moyen commandant les pulsations, tel une bascule ou "trigger", peut attaquer le circuit aux lieu et place de l'onde avec une fréquence variable de soixante à cent vingt hertz.

Le procédé de régulation se déroule donc en trois phases:

— à partir d'une pression choisie dite de consigne, l'appareil augmente progressivement le débit de pompe jusqu'à obtention de la pression de consigne au niveau de l'oreillette gauche et le microprocesseur calcule alors le rapport débit de pompe sur la pression auriculaire gauche et se sert de cette constante pour assurer la régulation,

— le débit de la pompe est rendu proportionnel au produit de la pression auriculaire gauche lue par la constante calculée et le débit de la pompe d'assistance suit donc directement la valeur de la pression auriculaire gauche lue par le capteur pour compenser immédiatement le déficit de la fonction pompe du coeur et pour, si au cours de la régulation la valeur de la pression de consigne est changée, calculer une pente, le dispositif se comportant comme un barorécepteur,

— s'il y a récupération du ad integrum du coeur, le sevrage se fait automatiquement par une décrementation de cent millilitres heure du débit de pompe qui se poursuit par palier jusqu'à obtention d'une nouvelle valeur de pression auriculaire gauche donnée, voire si elle n'est pas atteinte jusqu'à l'arrêt de la pompe, tandis que lorsque cette pression est atteinte, une alarme sonore se déclenche et le débit retombe sur la valeur antérieure pour ne recommencer le test de décrémentation que l'heure suivante avec éventuellement une nouvelle pression auriculaire gauche.

Dans le système pulsé, après lecture et détection du QRS d'électrocardiogramme, en phase diastolique, le débit est pulsé par accélération de la pompe, par l'intermédiaire, si le coeur est en fibrillation ventriculaire, d'un trigger interne avec fréquence fixe en réglant éventuellement le front de montée et le front de descente par rapport à l'électrocardiogramme ainsi que l'amplitude de la pulsion et de la durée.

## Revendications

1. Dispositif (21) de régulation de la vitesse et donc notamment du débit d'au moins une pompe (7, 7a) d'un système d'assistance d'un organe vivant, tel le muscle cardiaque, lequel système comprend principalement, au moins un circuit (3, 17) relié par ses extrémités (1 et 14 ou 16 et 18) à la sortie (2) et à l'entrée (15) du dit organe et, intercalée sur ce circuit (3, 17) la dite pompe (7, 7a) par exemple à galets (8, 8a), commandée par un moteur à vitesse variable, lequel dispositif comprend:
— des moyens de mesure (10, 11, 24) et d'enregistrement des conditions réelles de fonctionnement,
— des moyens d'élaboration et d'enregistrement des valeurs des consignes correspondantes de fonctionnement,
— des moyens de comparaison (29) de chacune des valeurs réelles à la valeur de consigne correspondante et d'élaboration d'un signal proportionnel à l'écart entre ces valeurs,
— des moyens d'élaboration, en fonction de ce signal proportionnel du signal déterminant la vitesse de la pompe,
— des portes de contrôle du seuil de chacun de ces signaux proportionnels et,
— des alarmes commandées chacune par l'un des signaux proportionnels lors de leur franchissement du seuil fixé,
ce dispositif étant caractérisé en ce qu'au moins l'un des moyens de mesure des conditions réelles de fonctionnement consiste en un moyen de mesure de pression.

2. Dispositif selon la revendication 1 caractérisé en ce qu'au moins l'un (24) des moyens de mesure de pression (10, 11, 24) comprend un élément de prélèvement de l'information pénétrant à l'intérieur de l'organe vivant à assister.

3. Dispositif selon la revendication 2 caractérisé en ce qu'en plus des moyens élaborant, au moins en fonction de la pression dans l'organe, le signal proportionnel déterminant la vitesse de la pompe du premier circuit, le dit dispositif comprend un amplificateur à gain variable par un potentiomètre et qui, à partir de ce signal, élabore un second signal semblable au premier dans des proportions réglables et apte à piloter la pompe (7a) d'un deuxième circuit (17) d'assistance d'un organe dans lequel le débit est physiologiquement proche sinon identique au premier.

4. Dispositif selon l'une quelconque des revendications 1 à 3 caractérisé en ce qu'il comprend un moyen de surmodulation, du signal déterminant la vitesse de la pompe.

5. Dispositif selon l'une quelconque des revendications 1 à 4 caractérisé en ce qu'il relie au circuit un moyen (6) de détection de bulles.

6. Dispositif selon l'une quelconque des revendications 1 à 5 caractérisé en ce qu'il comprend des moyens modifiant automatiquement et progressivement le signal déterminant la vitesse de la pompe dans le sens d'une décrémentation de cette vitesse et donc du débit et des moyens qui, en cas de pression dans l'organe traduisant l'échec du sevrage arrêtent immédiatement la décrémentation et rétablissent un signal déterminant le retour à une vitesse précédente.

7. Système d'assistance d'un organe vivant tel le muscle cardiaque comprenant principalement, au moins un circuit (3, 17) relié par ses extrémités (1 et 14 ou 16 et 18) à la sortie (2) et à l'entrée (15) du dit organe et, intercalée sur ce circuit (3, 17) la dite pompe (7, 7a) par exemple à galets (8, 8a), commandée par un moteur à vitesse variable, ce système étant caractérisé en ce qu'il comprend un dispositif de régulation selon l'une quelconque des revendications 1 à 6.

8. Système selon la revendication 7 caractérisé en ce que, sur le circuit, est intercalé un filtre et en ce que les moyens (10, 11) du dispositif prélevant l'information sur la pression dans le circuit sont reliés à ce circuit en amont et en aval de ce filtre.

9. Système selon la revendication 7 ou 8 caractérisé en ce que sur le circuit est intercalé un détecteur de bulles et en ce que le dispositif comprend des moyens qui arrêtent la pompe du circuit dès la détection des dites bulles.

10. Système selon la revendication 8 ou 9 caractérisé en ce qu'en vue de faciliter la détection d'hyperpression par les moyens de mesure de pression situés en amont du filtre, le circuit présente en aval de la pompe une coudure (25).

## Patentansprüche

1. Vorrichtung (21) zur Steuerung der Geschwindigkeit und also insbesondere des Durchsatzes zumindest einer Pumpe (7, 7a) eines Assistenz-Systems eines lebenden Organs wie der Herzmuskel, wobei das System hauptsächlich einen Kreislauf (3, 17) aufweist, der mit seinen Enden (1 und 14 oder 16 und 18) mit dem Ausgang (2) und dem Eingang (15) besagten Organes verbunden ist, und in diesen Kreislauf (3, 17) besagte Pumpe (7, 7a) eingeschaltet ist, beispielsweise mit Reibrollen (8, 8a), die durch einen Motor mit variabler Geschwindigkeit gesteuert ist, wobei die Vorrichtung aufweist:
— Meßmittel (10, 11, 24) und zum Speichern der tatsächlichen Funktionsbedingungen,
— Mittel zum Verarbeiten und zum Speichern der entsprechenden Funktions-Einstellwerten,
— Mittel (29) zum Vergleich jedes reellen Werts mit einem entsprechenden Einstellwert und zur Verarbeitung eines zu dem Unterschied zwischen den beiden Werten proportionalen Signals,
— Verarbeitungsmittel als Funktion dieses proportionalen Signals eines weiteren Signals, das die Geschwindigkeit der Pumpe bestimmt,
— Steuertore für den Schwellwert eines jeden der proportionalen Signale und
— Warnrufe, von denen jeder durch einer der proportionalen Signale gesteuert wird beim Übersteigen der festgelegten Schwelle,
dadurch gekennzeichnet, daß zumindest eines der Meßmittel für die reellen Funktionsbedingungen aus einem Druckmeßmittel besteht.

2. Vorrichtung nach Anspruch 1, dadurch gekennzeichnet, daß zumindest eines (24) der Druckmeßmittel (10, 11, 24) aus einem Abgriffelement für Informationen besteht, das in das Innere des lebenden zu unterstützenden Organs eindringt.

3. Vorrichtung nach Anspruch 2, dadurch gekennzeichnet, daß außerdem die Mittel zumindest als Funktion des Drucks in dem Organ das proportionale Signal verarbeiten, welches die Geschwindigkeit der Pumpe des ersten Kreises bestimmt, wobei die Vorrichtung einen Verstärker mit durch ein Potentiometer einstellbarer Verstärkung aufweist und der, ausgehend von diesem Signal ein zweites, mit dem ersten Signal in regelbaren Bereichen vergleichbares Signal verarbeitet und die Pumpe (7a) eines zweiten Unterstützungskreises (17) für ein Organ steuern kann, in welchem der Durchsatz physiologisch nahe wenn nicht identisch zu dem ersteren ist.

4. Vorrichtung nach einem der Ansprüche 1 bis 3, dadurch gekennzeichnet, daß die Vorrichtung eine Vorrichtung zur Auf-/Übermodulation des die Geschwindigkeit der Pumpe bestimmendes Signal aufweist.

5. Vorrichtung nach einem der Ansprüche 1 bis 4, dadurch gekennzeichnet, daß der Schaltkreis ein Mittel (6) zum Nachweis von Blasen aufweist.

6. Vorrichtung nach einem der Ansprüche 1 bis 5, dadurch gekennzeichnet, daß sie Mittel, die automatisch und zunehmend das Signal modifizieren, welches die Geschwindigkeit der Pumpe bestimmt, und zwar im Sinne einer Abnahme dieser Geschwindigkeit und infolgedessen des Durchsatzes und Mittel aufweist, die im Fall des Drucks in dem Organ den Mißerfolg des Abtrennens interpretieren, sofort die Verkleinerung anhalten und ein Signal wiederherstellen, welches die Zurückkehr zu der vorhergehenden Geschwindigkeit bestimmt.

7. Unterstützungssystem für ein lebendes Organ wie der Herzmuskel, mit hauptsächlich einem Kreis (3, 17), der mit seinen Enden (1 und 14 oder 16 und 18) mit dem Ausgang (2) und dem Eingang (15) des besagten Organes verbunden ist, und in diesem Kreis (3, 17) besagte Pumpe (7, 7a), beispielsweise mit Reibrollen (8, 8a) eingeschaltet ist, die durch einen Motor mit variabler Geschwindigkeit gesteuert ist, wobei das System dadurch gekennzeichnet ist, daß es eine Vorrichtung zum Steuern gemäß einem der Ansprüche 1 bis 6 aufweist.

8. System nach Anspruch 7, dadurch gekennzeichnet, daß in den Kreis ein Filter eingeschaltet ist und daß die Mittel (10, 11) der Vorrichtung, die die Information über den Druck in dem Kreis abnehmen, mit dem Kreis stromaufwärts und stromabwärts von diesem Filter verbunden sind.

9. System nach Anspruch 7 oder 8, dadurch gekennzeichnet, daß in den Kreis eine Nachweisvorrichtung für Blasen eingeschaltet ist und daß die Vorrichtung Mittel aufweist, die die Pumpe des Kreises bei Nachweis der besagten Blasen anhält.

10. System nach Anspruch 8 oder 9, dadurch gekennzeichnet, daß mit Hinblick auf die Erleichterung des Nachweises eines Überdrucks durch die Mittel zum Messen des Drucks, die stromaufwärts des Filters angeordnet sind, der Schaltkreis stromabwärts von der Pumpe eine Kröpfung (25) aufweist.

**Claims**

1. A device (21) for regulating the speed and therefore notably the flow of at least one pump (7, 7a) of a system for aiding a living organ such as the cardiac muscle, which system chiefly comprises at least one circuit (3, 17) connected at its ends (1 and 14 or 16 and 18) to the exit (2) and to the entrance (15) of the said organ, and, interposed on this circuit (3, 17), the said pump (7, 7a), for example one with pulleys (8, 8a) controlled by a variable-speed motor, which device comprises:
— means for measuring (10, 11, 24) and recording actual operating conditions,
— means for processing and recording values of the corresponding operating instructions,
— means for comparing (29) each of the actual values with the corresponding preset value, and for processing a signal proportional to the difference between these values,
— means for processing, depending on this proportional signal, the signal determining the speed of the pump,
— gates for monitoring the threshold of each of these proportional signals, and
— alarms each controlled by one of the proportional signals when they overstep the fixed threshold,
this device being characterised in that at least one of the means for measuring the actual operating conditions consists in a means for measuring pressure.

2. A device according to Claim 1, characterised in that at least one (24) of the means for measuring pressure (10, 11, 24) comprises an element for extracting information which penetrates to the inside of the living organ to be aided.

3. A device according to Claim 2, characterised in that, in addition to the means which process, at least depending on the pressure in the organ, the proportional signal determining the speed of the pump of the first circuit, the said device comprises an amplifier with gain variable by a potentiometer and which, starting from this signal, processes a second signal similar to the first in regulable proportions and capable of driving the pump (7a) of a second organ aid circuit (17) in which the flow is physiologically close, if not identical, to the first.

4. A device according to any one of Claims 1 to 3, characterised in that it comprises a means for overdriving the signal determining the speed of the pump.

5. A device according to any one of Claims 1 to 4, characterised in that it connects to the circuit a means (6) for detecting bubbles.

6. A device according to any one of Claims 1 to 5, characterised in that it comprises means which modify automatically and progressively the signal determining the speed of the pump in the direction of a decrease of this speed and therefore of the flow, and means which, in the event of pressure in the organ, translate the failure of privation of aid and immediately stop the decrease and reinstate a signal determining the return to a previous speed.

7. A system for aiding a living organ such as the cardiac muscle, chiefly comprising at least one circuit (3, 17) connected at its ends (1 and 14 or 16 and 18) to the exit (2) and to the entrance (15) of the said organ, and, interposed on this circuit (3, 17), the said pump (7, 7a), for example one with pulleys (8, 8a) driven by a variable-speed motor, this system being characterised in that it comprises a regulating device according to any one of Claims 1 to 6.

8. A system according to Claim 7, characterised in that a filter is interposed on the circuit and in that the means (10, 11) of the device which extract information about the pressure in the circuit are connected to this circuit upstream and downstream of this filter.

9. A system according to Claim 7 or 8, characterised in that a bubble detector is interposed on the circuit and in that the device comprises means which stop the pump of the circuit as soon as the said bubbles are detected.

10. A system according to Claim 8 or 9, characterised in that, in order to facilitate the detection of excess pressure by the pressure-measuring means situated upstream of the filter, the circuit has an elbow joint (25) downstream of the pump.

Fig:-1

0 139 548

Fig.- 3

Fig.- 2

Fig:_4

32

33

34

31

28

29

30

Fig:_5

32    47    53

33    54

38    48
39
40    49
41
42    55
43
44    45

34

29

35    50
36    51    56
37    52

46

31

57

Fig:_6

77

74    62    63

29    75

64
65
66
67
68
69
70

76

73    72

71

Fig:_7

61    29    58    59

60

0 139 548

3

Fig: 8

0 139 548